# EUROPEAN PATENT SPECIFICATION

(11) **EP 2 128 261 B1**
(45) Date of publication and mention of the grant of the patent: **06.06.2012**
(21) Application number: 06828411.6
(22) Date of filing: 20.12.2006
(51) Int. Cl.: C12N 15/861, C12N 7/00, A61K 35/76, A61K 39/235, A61K 48/00, A61P 35/00, C12R 1/92, C07K 14/47

(54) **A RECOMBINANT ADENOVIRUS COMPRISING RECOMBINANT KHP53 GENE AND PREPARATION METHOD AND USES THEREOF**
REKOMBINANTER ADENOVIRUS MIT REKOMBINANTEM KHP53-GEN UND HERSTELLUNGSVERFAHREN UND VERWENDUNGEN DAFÜR
ADÉNOVIRUS RECOMBINANT COMPRENANT UN GÈNE KHP53 RECOMBINANT ET SON PROCÉDÉ DE PRÉPARATION ET SES UTILISATIONS

(43) Date of publication of application: 02.12.2009
(73) Proprietor: Shenzhen Chingen Genetic Engineering Co., Ltd., Shenzhen City, Guangdong Province (CN)
(72) Inventor: XIE, Qingjun, Guangdong 518057 (CN)
(74) Representative: Schumacher & Willsau
(86) International application number: PCT/CN2006/003506
(87) International publication number: WO 2008/074189

(56) References cited:
- EP-A1- 1 308 517
- WO-A2-00/52187
- WO-A2-03/050249
- WO-A2-2006/063082
- CA-A1- 2 246 626
- CN-A- 1 952 160
- US-A1- 2006 063 259
- KOZAK M: "AN ANALYSIS OF 5'-NONCODING SEQUENCES FROM 699 VERTEBRATE MESSENGERRNAS", NUCLEIC ACIDS RESEARCH, OXFORD UNIVERSITY PRESS, SURREY, GB, vol. 15, no. 20, 1 January 1987 (1987-01-01), pages 8125-8132, XP002065806, ISSN: 0305-1048
- MATTHEWS D A ET AL: "Development and use of a 293 cell line expressing lac repressor for the rescue of recombinant adenoviruses expressing high levels of rabies virus glycoprotein", JOURNAL OF GENERAL VIROLOGY, vol. 80, no. 2, February 1999 (1999-02), pages 345-353, XP002612184, ISSN: 0022-1317
- "AD-P53, AD5CMV-P53, ADENOVIRAL P53, INGN 101, P53 GENE THERAPY - INTROGEN RPR/INGN 201", BIODRUGS: CLINICAL IMMUNOTHERAPEUTICS, BIOPHARMACEUTICALS AND GENE THERAPY, ADIS INTERNATIONAL, FR, vol. 17, no. 3, 1 January 2003 (2003-01-01), pages 216-222, XP009074513, ISSN: 1173-8804, DOI: DOI:10.2165/00063030-200317030-00010
- ROTH JACK A: "Adenovirus p53 gene therapy", EXPERT OPINION ON BIOLOGICAL THERAPY, ASHLEY, LONDON, GB, vol. 6, no. 1, 1 January 2006 (2006-01-01) , pages 55-61, XP009087946, ISSN: 1471-2598, DOI: DOI:10.1517/14712598.6.1.55
- DATABASE BIOSIS [Online] BIOSCIENCES INFORMATION SERVICE, PHILADELPHIA, PA, US; February 2008 (2008-02), XIE QING-JUN ET AL: "Construction and expression of recombinant high IQ adenovirus vector containing human p53 gene leaded by Kozak sequence", XP002612185, Database accession no. PREV200800366528

## Description

### Field of the invention

The invention belongs to the field of genetic engineering. In particular, the invention relates to a recombinant virus vector for a human tumor suppressor gene, namely to a Kozak sequence-directed recombinant adenovirus vector for a human p53 tumor suppressor gene, and also to a pharmaceutical composition comprising said recombinant adenovirus vector. The invention further discloses the preparation methods and uses of the recombinant adenovirus vector.

### Background of the Art

### 1. Tumor gene therapy

Gene therapy is a molecular medical therapy aiming for radically curing diseases, which comprises the following steps: introducing a gene or DNA (RNA) fragment with therapeutic function into human target cells in a certain way; and modulating the expression of the gene at a molecular level in accordance with various pathological changes resulted in diseases, so as to exert the therapeutic function.

Tumor gene therapy has been rapidly developed for the past more than 10 years. In the United States, there founded 10 large gene therapy companies in succession around 1995. The number of professional gene therapy companies reached about 30 in 1999, and almost 100 in 2000. In 2004, among the 240 or more gene therapy companies in the whole world, 75 of them were researching and developing tumor gene therapies. Until the end of July 2004, the number of approved clinical experimental procedures of gene therapies reached 987 in 23 countries in the world. Most of those procedures (656 of the 987 procedures) , accounting for 66.5%, targeted at tumors. The administration routes included intra-tumor tissue, intravenous, intra-hepatic artery, subcutaneous, intramuscular, intra-bladder, and intraperitoneal, etc. The first commercially available gene therapeutic medicine in the world was used to treat tumors.

The research and application of a gene therapy is derived from the treatment of genetic diseases, but the tumor gene therapy has been developed far beyond genetic diseases therapy recently. The major reason is that the incidence of tumors and the number of patients with tumors are much greater, while compared with those of genetic diseases. According to the report by the World Health Organization, the number of patients with newly diagnosed malignant tumors was 10.1 millions in 2000, wherein 0.62 million patients died. 22.4 million patients are suffering from malignant tumors currently. During the past 10 years, the global morbidity of malignant tumors was increased 22%, which indicated that the malignant tumor had become one of the major common diseases severely shortening human lives and lowering life quality. The malignant tumors not only dramatically impact human health, but also bring significant mental and physical harm to patients. A secondary reason is that there exists a large amount of widely ranged therapeutic gene types, which are not only restricted within defected or mutated genes as in genetic diseases. Any gene, such as a tumor suppressor gene, an immune enhancer gene, a suicide gene, a neovascularization suppressor gene, etc can be utilized, as long as it enables the treatment purpose of tumors but only slightly hurts normal somatic cells. The third reason is that the gene therapy of tumors does not require constant expression of ectogenic genes. Periodical expression or even temporary expression of the gene can kill tumor cells as well. The final reason is that most of the gene therapies of tumors involve introducing therapeutic genes into tumor cells directly, which requires less accuracy for modulating the expression of therapeutic genes than genetic diseases does.

### 2. Investigation on human p53 gene

Lane and Crawford et al. (1979) (Lane D P, Crawford L V.T antigen is bound to a host protein in SV40 transformation cells[J].Nature , 1979 / 278 / 570 P 261-263) found a p53 gene in the cell transfected by a.SV40 large T antigenic gene, and believed it was an oncogene. After that, through studies, Finlay et al. (Levine AJ, Momand J, Finlay CA. The p53 tumor suppressor gene [J]. Nature, 1991, 351(3):453) discovered that if there existes a wild type p53 gene in the cell transfected by an oncogene such as myc or ras, the growth of said cell would be inhibited. Therefore, it is suggested that the p53 gene is a cancer suppressor gene.

Human p53 gene is located at 17p13.1 region of the 17^{1h} chromosome, with a length of 20303bp in the genomic DNA. Human p53 gene comprises 11 exons and 10 introns, which encodes a protein of 393 amino acid residues, i.e. P53 protein. According to the functions, P53 protein is divided into 5 parts: N-terminal transcription activation region (1-42 aa), signal region (64-92 aa), DNA binding region (102-292 aa), tetramer region (324-355 aa), and C-terminal non-specific DNA binding region (370-393 aa). The N-terminal transcription activation region can bind to a series of protein structures (such as MDM2 TBP TAFs), thereby adjusting the transcription function of p53. The signal region enriches proline, which comprises 5 repeats of proline-X-X-proline, this region is not necessary for the transcription activation, but it is essential for inducing apoptosis. The DNA binding region recognizes sequence-specific DNA sequences. The tetrame region can inhibit the growth of cancer cells. The C-terminal non-specific DNA binding region can bind to non-specific DNA sequences. The basic function of p53 gene is acting as a nucleic transcription factor. The anti-tumor bioactivity thereof mainly includes blocking tumor cell cycles such as G1/S and G2/M; inducing tumor cell apoptosis; suppressing the angiogenesis of tumors; enhancing the immunogenicity of tumor cells; inhibiting the infiltration and metastasis of tumor cells; and raising the sensitivity of tumor cells to radiochemical therapies and thermotherapies, etc.

p53 gene mutation is one of the major reasons for the genesis of many tumors. The gene mutation mainly includes point mutation, allelic deletion, gene rearrangement, and inactivation by binding to tumor virus oncoproteins, etc. It was reported that p53 gene mutation happened in 50% of the approximately over 200 different types of tumors. It has been disclosed that there are 4 mutation hotspots located at exons 5-8 in p53 gene. Although the mutation spectra of p53 gene in tumors generated in different tissue organs are different, around 90% mutations focus in those hotspots. Such hotspots encode amino acid sequences at position 132-143, 174-179, 236-248 and 272-281, respectively.

In many types of human tumors, for example in oesophageal cancer, lung cancer, gastric cancer, liver cancer, colonal and rectal cancer, pancreatic cancer, breast cancer, ovarian caner, thyroid cancer, osteosarcoma, rhadomyosarcoma, neuroblastoma, about 50% p53 genes mutate. In melanoma (primary), the mutation rate of p53 gene is up to 97%. p53 gene mutation occurs in all types of lung cancer. For instance, p53 gene mutation occurs in about 52% of non-small cell lung carcinoma and 90% of small cell lung carcinoma. p53 gene also mutates in some leukaemia, cerebroma, multiple myeloma, astrocytoma. Studies showed that p53 mutation rate was higher in refractory tumors and lower in relatively tractable tumors. Moreover, p53 gene mutation closely relates to the progress of diseases, metastasis of tumors and the sensitivity and prognosis of radiochemical therapies.

### 3. Investigation on adenovirus vector

The structure of adenovirus is the characteristic icosahedron capsid, with no envelope, a diameter of 70~90nm, and 252 capsomers, in which the icosahedron parietal angled capsomers are 12 pentons with 2 fibers each. Besides the pentons, there also exists 240 non-parietal angled capsomers, called hexons. The genome of adenovirus is a linear double-stranded DNA of ~36kb binding with a terminal protein (TP) covalently at its 5' -end. There are also inverted terminal repeats (ITRs) located at the 5'-end. Virus DNA closely binds to core protein VII and a small peptide called mu. Another protein V coats the resultant DNA-protein complex, and protein VI provides a structural linkage between the DNA-protein complex and the capsid. To prepare an adenoviral genome that acts as a gene therapeutical vector, its E1 region and/or E3 region are usually deleted, and an exogenous gene expression cassette is added into the position of the E1 region. The resulted recombinant adenovirus is a replication-defective virus, which means it can not be replicated in a normal cell, but can only be replicated and generated in a specific cell (such as 293 cell).

The characteristics of an adenovirus vector include: larger capacity of vectors C > 7.5kb), extremely high transfection rate (the *in vitro* transfection rate of human tumor cells reaches 100%), high productive efficiency (viruses can be amplified hundreds of times by producing cells), non-integrative infection of cells, genetic non-toxicity, strong infecting ability (both cleavage and non-cleavage cells may be infected), high expression of an exogenous gene. Those characteristics enable adenoviruses to be especially suitable for gene therapy of tumors and industrial production of the products thereof.

The evolvement of the method for preparing the recombinant adenovirus vector strain includes: two-plasmid co-transfection method, Ad-Easy method, and AdMax method. The two-plasmid co-transfection method is a less efficient method used in an earlier stage, which comprises the following steps: co-tranfecting packaging cells, 293 cells with a shuttle vector small plasmids carrying a target gene and a large plasmid carrying the adenoviral genome; generating the genome of recombinant adenovirus vector by random homologous recombination of both said plasmids in the 293 cells; and packaging into the virions in the 293 cells. Ad-Easy method is a more efficient method, comprising the following steps: transforming bacteria carrying most parts of the adenoviral genome with a adenovirus shuttle plasmid carrying a cloned exogenous gene; generating a plasmid containing the recombinant adenovirus vector genome by recombination under the action of bacterial recombination enzyme Cre; amplifying and extracting the plasmid after screened for resistance; transfecting 293 cells with the plasmid after digested and purified; and packaging into the recombinant virons in the 293 cells. AdMax method is the method with the highest packaging efficiency so far, which comprises the following steps: co-transfecting 293 cells with an adenovirus shuttle plasmid carrying a cloned exogenous gene and a packaging plasmid carrying the most parts of adenoviral genome; and generating the recombinant adenovirus by recombination under the action of recombination enzymes (Ng P, Parks RJ, Cummings DT, et al. An enhanced system for construction of adenoviral vectors by the two-plasmid rescue method [J]. Hum Gene Ther. 2000,11 (5): 693-699.).

The expression of an adenovirus vector carrying an exogenous gene often results in a certain level of cytotoxicity. Sometimes, even a medium expression level of a gene without cytotoxicity may clearly block the replication and amplification of an adenovirus vector. As a result, it is very difficult to create a recombinant adenoviron with transfective activity, or to amplify it to obtain a high titer value. In such circumstances, the virus highly expressing a target protein can not be obtained by the virus monoclonal screening method either. For example, the adenovirus expressing a glycoprotein is usually difficult to produce virion, and so are some adenovirus vectors expressing a certain DNA binding protein or enzyme. Therefore, it is hard to estimate the titer value and the yield of a certain adenovirus strain in advance.

The preparation of the adenovirus vectors described above can be achieved by AdMax™ Hi-IQ adenovirus vector system. Based on AdMax, AdMax™ Hi-IQ adenovirus vector system utilizes a lac operator to control the transcription of target genes, and packages adenovirus vectors on the 293 cells expressing lac repressors, thereby inhibiting the expression of the target genes on those cells. However, the expression of target genes on other cells is not inhibited.

"Hi-IQ" system can be used for the high yield preparation of an adenovirus vector carrying a target gene providing or potentially providing cytotoxicity. Unlike AdMax system, "Hi-IQ" system includes a 293 cell line expressing a lac repressor (293-IQ cell) and a shuttle plasmid containing a MCMV promoter, an intron and a lac operator, and a helper large plasmid containing the adenoviral genome. The resultant recombinant virus is an E1/E3 deleted replication-defective adenovirus.

The recombinant human p53 adenovirus has been applied to clinically treat malignant tumors for ten years. Although some exciting therapeutic effects have been achieved, in general, the therapeutic effects are still uncertain. The major reason for that is a relative lack of P53 protein expression in tumor cells, which is due to the common utilization of a weaker promoter such as RSV, SV40 or CMV in the eukaryotic expression system of p53 tumor suppressor gene. Some studies indicated that when gene mutation occurred in a normal human cell under various *in vivo* or *in vitro* conditions and finally caused the formation of a tumor cell, more than 10000 alternation at a gene level (such as deletion, point mutation and methylization of cancer suppressor gene, and rearrangement, amplification and mutation of pro-oncogene, etc) had been accumulated. However, the action mechanism of tumor suppressor gene p53 is to repress the growth of tumors by blocking cell cycles, facilitating apoptosis of tumor cells, inducing differentiation and aging of cells, and inhibiting angiogenesis in tumors, etc. Therefore, the most significant action feature of p53 is multiple target points, i.e. not only one single action point. For instance, P53 protein, as a transcription factor, directly reacts with a specific DNA sequence, thus regulating the expression of various genes downstream. A genechip research showed that the transcription of 107 genes was activated by P53 protein and thereby the expression thereof was up-regulated, whereas the expression of 54 genes was down-regulated due to the inhibition for their transcription. Also, P53 protein *per se* can interact with several intracellular proteins. For example, the gene expression product of oncogene mdm2 can specifically bind to P53 protein, thereby inhibiting the function of P53. Accordingly, unlike the plenty of gene changes in tumor cells, after the recombinant human p53 adenovirus gene medicine formulation, used for gene replacement therapy, is utilized to infect tumor cells and is expressed, the expressed amount of P53 protein is little, thus it is difficult to effectively control tumor growth.

However, after we constructed the human p53 recombinant adenovirus shuttle vector with high expression ability, it was found that such a vector was not able to package into a recombinant adenovirus in 293 cells, and thereby to transduct a human p53 gene into a tumor cell, thereby it could not be used to achieve the purpose of a gene therapy. The major reason for that is the equally effective expression of the expression system highly expressing human p53 gene in the packaging cells depletes a large amount of resources used for the protein synthesis in packaging cells (because the packaging process of a recombinant adenovirus is also a process in which a large amount of proteins are synthesized, there forms a competitive relation between them). Moreover, many P53 proteins result in blocking cell cycles of 293 cells, or even apoptosis. Hence, besides the effective expression of genes, the effective packaging of a recombinant adenovirus is also needed.
US 2006/0063259 A1 discloses methods, cells and recombinant adenoviral vectors that permit the production of recombinant adenoviral vector stocks with reduced levels of contamination by replication competent adenoviruses.
WO 03/050249 A2 provides a system for producing pancreatic islet cells from embryonic stem cells. In this context, the Ad shuttle vector pDC515 comprising neurogenin 3 is inserted downstream of the MCMV promoter, whereby the sequence upstream of the ATG start codon was replaced with the consensus Kozak sequence.
Kozak M: "An analysis of 5'-noncoding sequences from 699 vertebrate messenger RNAs", NUCLEIC ACIDS RESEARCH vol.15, no 20, 1 January 1987, pages 8125 - 8132 describes 5'-noncoding sequences that have been compiled from 699 vertebrate mRNAs.
WO 00/52187 teaches a way to construct viruses and plasmids which contain viral DNA.
Matthews D et. al.: "Development and use of a 293 cell line expressing lac repressor for the rescue of recombinant adenoviruses expressing high levels of rabies virus glycoprotein", JOURNAL OF GENERAL VIROLOGY, vol. 80, no. 2, February 1999, pages 345 - 353, provides a cell line that contains and expresses a derivation of the lac repressor protein
WO 2006/063082 A2 relates to methods an compositions for including apoptosis in cancer cells and for identifying drugs that modulate apoptotic path ways.
"AD-P53, AD5CMV-P53, Adenoviral P53, INGN 101, P53 gene therapy - INTROGEN RPR/INGN 201", BIODRUGS: CLINICAL IMMUNOTHERAPEUTICS, BIOPHARMACEUTICALS AND GENE THERAPY vol. 17, no. 3, 1 January 2003 (2003-01-01), pages 216-222 and ROTH JACK A: "Adenovirus p53 gene therapy", EXPERT OPINION ON BIOLOGICAL THERAPY, ASHLEY, LONDON, GB, vol. 6, no. 1, 1 January 2006 (2006-01-01), pages 55-61 show that Ad vectors are routinely used for cancer gene therapy.

### Disclosure of invention

The invention is as defined in the claims and provides a recombinant adenovirus, wherein multiple regulation elements improving gene expression, such as MCMV(Murine Cytomegalovirus) promoters, introns, and Kozak sequences, etc, are used comprehensively in an eukaryotic expression system inserted in said recombinant adenovirus, leading human p53 tumor suppressor gene to enter a tumor cell through a recombinant adenovirus vector at different levels and stages of gene expression, directly followed by the mass expression of p53 in the tumor cell, inhibition for the growth of the tumor cell, and apoptosis in the end. Also, an *E. coli* Lac Operator is inserted into the downstream of MCMV in the eukaryotic expression ssytem, and a lac repressor gene specifically binding to the DNA sequence of *E. coli* Lac Operator is transferred into 293 packaging cells and expressed stably. In this way, in the packaging process of the recombinant adenovirus, the lac repressor protein expressed in 293 cells is located between MCMV promoter and human p53 tumor suppressor gene, which disables the normal transcription of p53 gene, and clearly represses the expression of an exogenous gene in 293 cells, but does not affect the packaging process of the recombinant adenovirus. The above two methods permit human p53 tumor suppressor gene to be effectively expressed in tumor cells and to be effectively packaged in the transgenetically engineered 293 cells (293IQ cells).

Accordingly, one aspect of the invention relates to a recombinant adenovirus vector, wherein a Kozak sequence and an exogenous gene are inserted between the operator of the adenovirus shuttle vector pDC315(io) or pDC316(io) and a polyA signal sequence.

Another aspect of the invention relates to a recombinant adenovirus vector, wherein a Kozak sequence and an exogenous gene are inserted between the operator of the adenovirus shuttle vector pDC515(io) or pDC516(io) and a polyA signal sequence.

Said Kozak sequence is CCACCATGG; said exogenous gene is human p53 gene,the sequence thereof is as defined by SEQ ID NO:3.

A further aspect of the invention relates to an eukaryotic gene expression system, comprising the sequence of SEQ ID NO:11. Preferably, the sequence thereof is shown as SEQ ID NO:11.

A further aspect of the invention also relates to a recombinant adenovirus, which is obtained by co-transfecting 293IQ cells with said recombinant adenovirus shuttle vector and a adenovirus backbone, wherein when said adenovirus shuttle vector is pDC315(io) or pDC316(io), said adenovirus backbone is pBHGlox(delta)E1,3Cre; when said adenovirus shuttle vector is pDC515(io) or pDC516(io), said adenovirus backbone is pBHGfrt(del)E 1,3FLP.

Preferably, said recombinant adenovirus is an adenovirus that was deposited in China Center for Type Culture Collection on June 20^{th}, 2005, with a deposit number of CCTCC V200508.

Another aspect of the invention relates to a medicament for use in treating cancers, said medicament comprises the recombinant adenovirus of the present invention and optionally, a pharmaceutically acceptable excipient.

A further aspect of the invention also relates to use of said recombinant adenovirus for the manufacture of a medicament for treating cancers, wherein said cancer is selected from lung cancer, liver cancer, breast cancer, nasopharyngeal cancer, ovarian caner or cervical cancer.

The present invention also relates to a method for preparing said recombinant the defined p53 the defined adenovirus vector, comprising the step of inserting the defined p53 gene linked to the defined Kozak sequence between the operator of adenovirus shuttle vector pDC315(io), pDC316(io), pDC515(io) or pDC516(io) and a polyA signal sequence in a forward direction.

In addition, the invention also relates to a method for obtaining said recombinant adenovirus, comprising the step of co-transfecting 293IQ cells with the recombinant adenovirus vector and an adenovirus backbone, wherein when said adenovirus shuttle vector is pDC315(io) or pDC316(io), said adenovirus backbone is pBHGlox(delta)E1,3Cre; when said adenovirus shuttle vector is pDC515(io) or pDC516(io), said adenovirus backbone is pBHGfrt(del)E1,3FLP.

In another aspect, there is also described a method for treating cancers, comprising the step of administering a therapeutic effective dose of the recombinant adenovirus of the invention to a patient. The therapeutic effective dose is conveniently determined by one skilled in the art or a physician in accordance with particular circumstances. Particularly, the invention relates to the following steps: amplifying the coding region of human p53 tumor suppressor gene by PCR with 5'-ATA GGATCC ACCATGG AGG AGC CGC AGT C 3' (SEQ ID NO:1) as a upstream primer and 5'-ATA GGATCC ATG TCA GTC TGA GTC AG 3' (SEQ ID NO:2) as a downstream primer; cloning the PCR product into a T vector followed by sequencing in both directions; sub-cloning it into a adenovirus shuttle plasmid (pDC315(io), pDC316(io), pDC515(io) or pDC516(io)) using restriction endonulease BamH I; screening a clone inserted in a forward direction by PCR; co-transfecting 293IQ cells with the clone and the DNA of a adenoviral genomic backbone plasmid (pBHGlox(delta)E1,3Cre or pBHGfrt(del)E1,3FLP); packaging a recombinant adenovirus, i.e. rAdMH-khp53 by site-specific homologous recombination in eukaryotic cells; cloning a positive recombinant adenovirus strain; infecting 293IQ cells by the cloned positive recombinant adenovirus strain; amplifying and purifying; and manufacturing medicines for a gene therapy.

The features of the recombinant adenovirus include:
1. A unique eukaryotic gene expression system structure
   First of all, the currently most effective promoter for promoting eukaryotic gene expression in the world, i.e. Murine Cytomegalovirus promoter MCMV, is used. Therefore, gene expression is improved at transcription level. An intron is inserted between the promoter and an exogenous gene, which further increases the stability of mRNA after gene transcription. Secondly, there is a delicately designed Kozak sequence located before the coding region of human p53 tumor suppressor gene, thus further raising the expression amount at protein translation level. Lastly, *E. coli* lac operator is inserted into the downstream of MCMV. The co-function of *E. coli* lac operator and a 293IQ cell effectively facilitates the eukaryotic gene high expression system to be recombined into an adenovirus vector and to be packaged.
2. The advantages of an adenovirus vector include: high transfecting rate, excellent maneuverability, ability to carry larger genes, capability of preparing an effective viron with higher titer, a wide range of hosts, great safety, and low pathogenicity.
3. The multiple targeting property of an anticancer function of human p53 tumor suppressor gene not only blocks tumor cell cycles, represses the growth of tumor cells, and induces apoptosis of tumor cells, but also induces differentiation and aging of cells, suppresses angiogenesis of tumors, enhances the immunogenicity of tumor cells, inhibits the infiltration and metastasis of tumor cells; and improves the sensitivity of tumor cells to radiochemical therapies and thermotherapies, etc.
4. Human p53 tumor suppressor gene is introduced into a human tumor target tissue cell by adenovirus vector and expressed directly in that cell, which overcomes the difficulty that P53 protein is unable to be prepared into a recombinant genetic engineering product *in vitro* by a genetic engineering method, because of its instability. Moreover, the introduction and expression of human p53 tumor suppressor gene by adenovirus vector makes no difference with an endogenous P53 protein in modification procedures of protein molecules, such as protein phosphorylation, folding and polymerizing, etc, and brings about the exactly same bioactivity, so as to directly exert the anticancer function for topical tumors.

It is contributed by the invention that the constructed recombinant adenoviruses with the unique structures and features, for example rAdMH-khp53, realize the effective expression of human p53 tumor suppressor gene in a human tumor target tissue cell and the effective packaging of human p53 tumor suppressor gene in a specific 293IQ cell. Therefore, the anticancer function thereof is clearly better than that of some low expression systems, which enables the tumor cells insensitive to a low p53 expression gene therapy to be equally effectively inhibited. Therefore, the treatment becomes more accurate and useful. The inventive human p53 tumor suppressor gene linked to an adenovirus can be expressed directly in an eukaryotic cell, meaning that it can be introduced directly into a solid tumor site and expressed in such a site. That makes the subjects receiving the treatment to become a "factory" for producing P53 tumor suppressor proteins. Such a method is able to overcome some disadvantages, such as instability of proteins, short half-life, low activity, and high cost, etc. Therefore, said treatment becomes a therapy acceptable and affordable by most patients.

The 293IQ cell used in the experiments is obtained by introducing an *E*. *coli* lac suppressor gene into a 293 cell. Detailed information refers to Matthews D A, Cummings D, Evelegh C, et al. Development and use of a 293 cell line expressing lac repressor for the rescue of recombinant adenoviruses expressing high levels of rabies virus glycoprotein. Journal of General Virology (1999), 80, 345-353.

### Brief description of the drawing

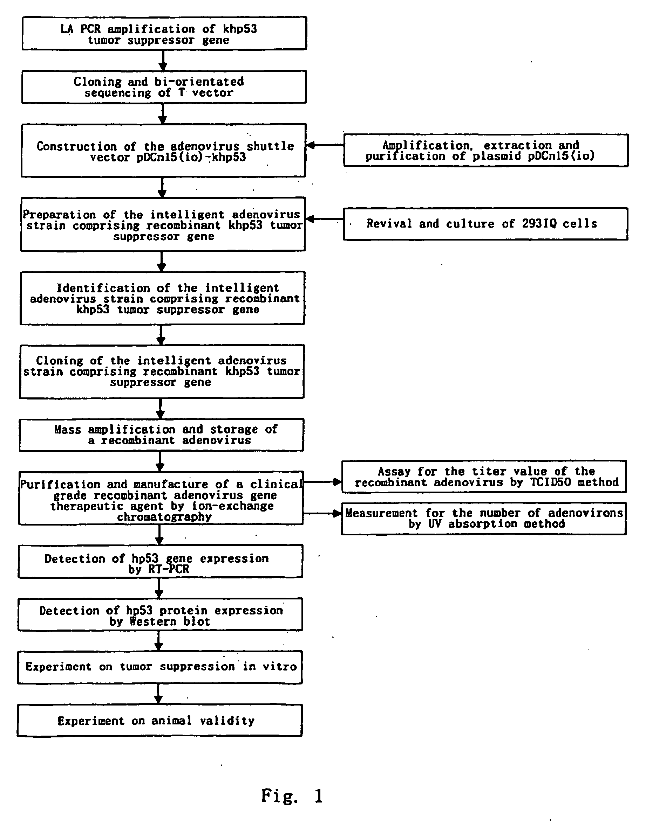

Figure 1 is a technique flow chart for investigation on the recombinant khp53 tumor suppressor gene adenovirus.
Figure 2 shows the result of an agarose gel electrophoretic analysis of the human p53 gene-coding fragment carrying a Kozak sequence, wherein M: DL2000 DNA Marker; 1: a target fragment of 1206bp; 2: a negative control.
Figure 3 indicates the result of digestion and electrophoretic identification of a recombinant plasmid, pDC515(io)-khp53, wherein lane M:Lamda/EcoT141 DNA Marker; lane 1: the recombinant plasmid, i.e. pDC515(io)-khp53; lane 2: pDC515(io)-khp53/BamHI; lane 3: pDC515(io)- khp53/EcoRI.
Figure 4 shows the formation of the recombinant adenovirus rAdMH-khp53 strain, wherein A: a negative control; B: the 293IQ cells with CPE; C: the 293IQ cells with complete CPE.
Figure 5 shows the result of PCR and electrophoretic identification of the recombinant adenovirus rAdMH-khp53, wherein lane M:DL2000 DNA Marker; lane 1: a negative control; lane 8: a positive control; lanes 2-7: the clones under identification.
Figure 6 is a schematic drawing of the genomic structure of the recombinant adenovirus rAdMH-khp53, wherein ① is an intron; ② is a MCMV promoter; ③ is an operator; ④ is the hp53 coding sequence; ⑤ is a Kozak sequence; ⑥ is SV40PolyA.
Figure 7 is the result of RT-PCR and electrophoretic analysis, showing the expression of p53 mRNA after infecting Saos-2 cells with rAdMH-khp53, wherein
   lane 1: the PCR amplification result (a positive control), wherein the template is the RT products of total RNA of A549 cells, the primers are the specific primers in p53 gene coding region (the same primers are used in lanes 2-4);
   lane 2: the PCR amplification result (a negative control), wherein the template is the RT products of total RNA of Saos-2 cells;
   lane 3: the PCR amplification result, wherein the template is the RT products of total RNA of Saos-2 cells infected with rAdMH-khp53;
   lane 4: the PCR amplification result without any template (a blank control);
   lane M: DL2000 DNA Marker;
   lane 5: the PCR amplification result without any template (a blank control), wherein the primers are GAPDH gene specific primers (the same primers are used in lanes 6-8);
   lane 6: the PCR amplification result, wherein the template is the RT products of total RNA of Saos-2 cells infected with rAdMH-khp53;
   lane 7: the PCR amplification result, wherein the template is the RT products of total RNA of Saos-2 cells;
   lane 8: the PCR amplification result, wherein the template is the RT products of total RNA of A549 cells.

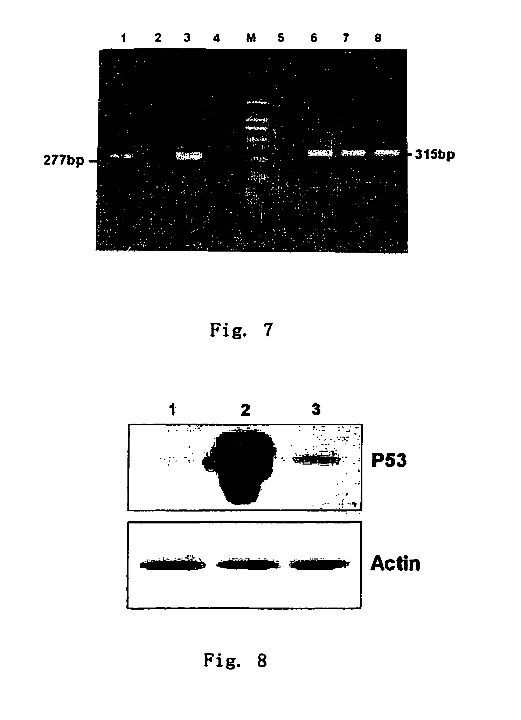

Figure 8 shows the Western blot result of Saos-2 cells which have been infected with rAdMH-khp53 for 10 hours, wherein lane 1: total proteins of Saos-2 cells; lane 2: total proteins of the Saos-2 cells infected with rAdMH-khp53; lane 3: total proteins of A549 cells.

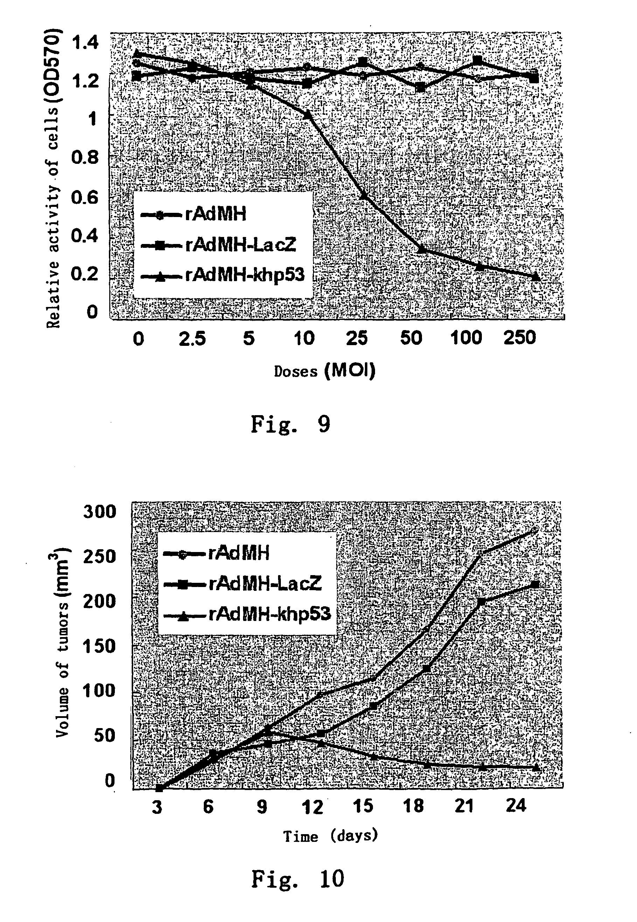

Figure 9 shows the anticancer activity of the recombinant adenovirus rAdMH-khp53 *in vitro.*
Figure 10 shows the anticancer activity of the recombinant adenovirus rAdMH-khp53 *in vivo.*

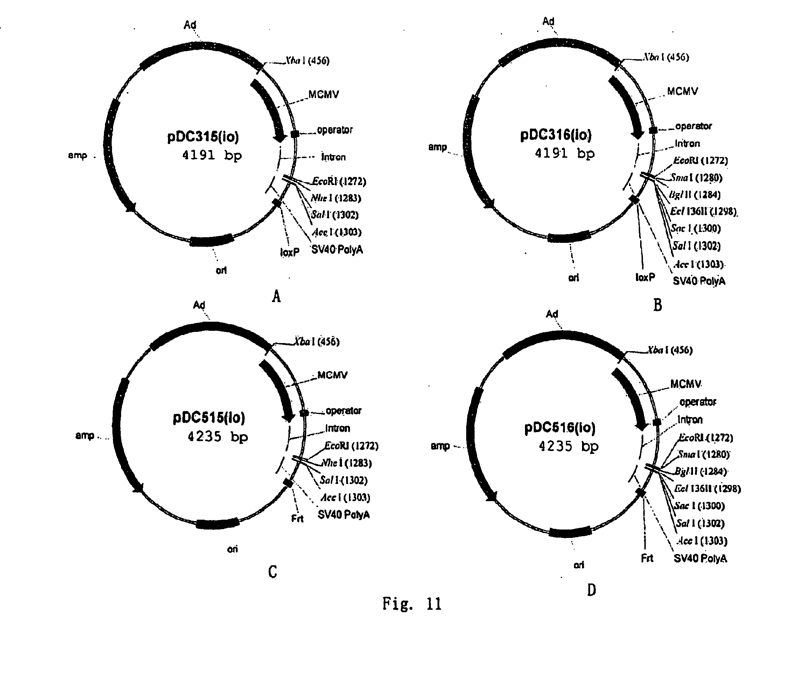

Figure 11 is a drawing showing the adenovirus shuttle vector, wherein A: pDC315(io), B: pDC316(io), C: pDC515(io), D: pDC516(io).

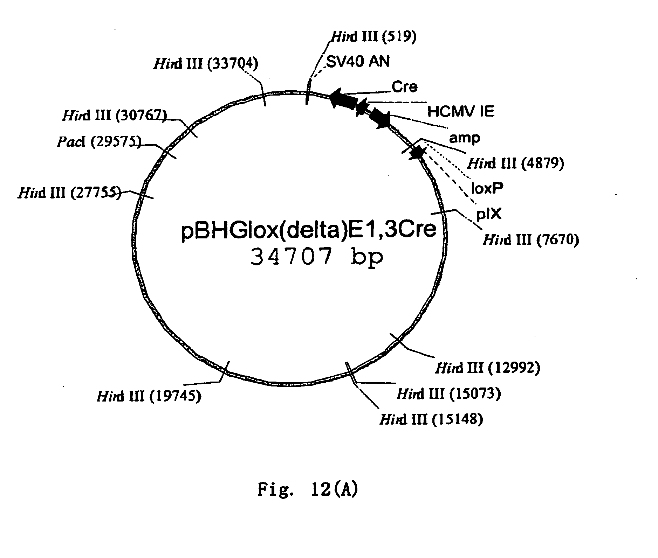

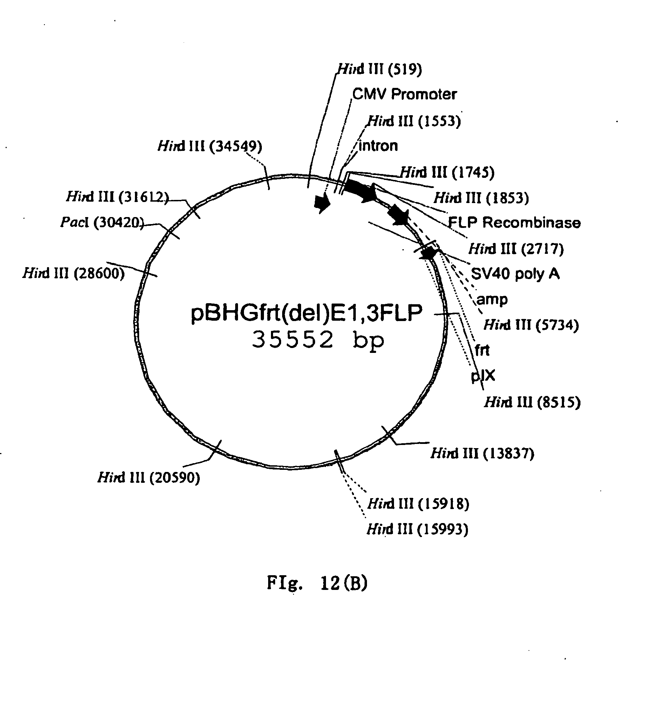

Figure 12 is a drawing showing the adenovirus backbone DNA, wherein A: pBHGlox(delta)E1,3Cre; B: pBHGfrt(del)E1,3FLP.

### Detailed embodiments of the invention

The present invention is further explained and illustrated by the following examples which are not to be construed to limit the scope of the invention.

The adenovirus vectors pDC315(io) and pDC316(io) used herein and the corresponding adenovirus backbone pBHGlox E1,3Cre were contained in a AdMax HI-IQ Kit H, commercially purchased from Pen Yuan Zheng Yang gene tech Ltd., with a catalog number of PD-01-68. The AdMax HI-IQ Kit H further comprises pFG 140 and 293IQ cells.

The adenovirus vectors pDC515(io) and pDC516(io) used herein and the corresponding adenovirus backbone pBHGfrt E1,3FLP were contained in a AdMax™HI-IQ Kit J, commercially purchased from Pen Yuan Zheng Yang gene tech Ltd., with a catalog number of PD-01-69. The AdMax™HI-IQ Kit J further comprises pFG140 and 293IQ cells.

### Example 1: Construction of the adenovirus recombinant comprising recombinant khp53 tumor suppressor gene

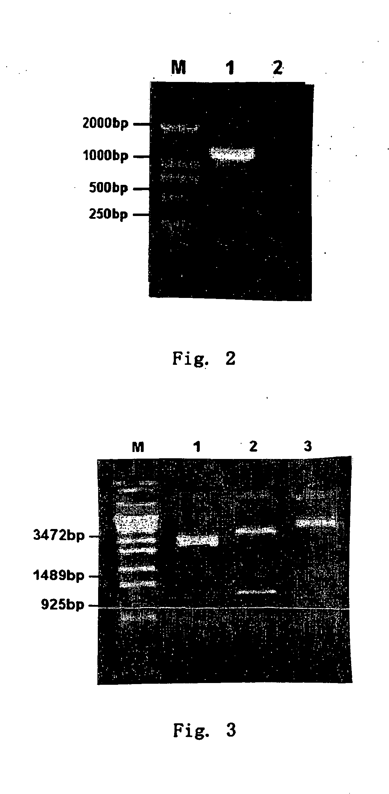

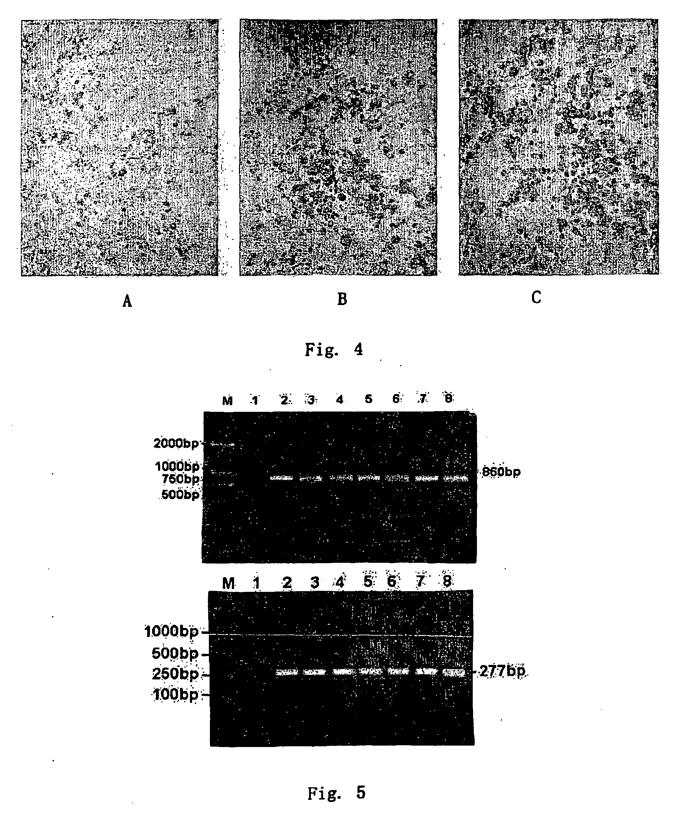

1) Obtaining human p53 tumor suppressor gene coding sequence carrying a Kozak sequence (shorten as khp53):
   Two primers were designed according to the published human wild-type p53 cDNA full sequence (SEQ ID No:3): a upstream primer 5' -ATA GGATCC *A CCATGG AGG AGC CGC AGT C* 3' (SEQ ID NO:1) and a downstream primer 5' -ATA GGATCC ATG TCA GTC TGA GTC AG 3' (SEQ ID NO:2). The BamHI digestion site GGATCC and the digestion protective bases ATA were inserted into both primers. The base A was inserted behind the BamHI digestion site GGATCC of the upstream primer, which is followed by the sequence *CCATGG AGG AGC CGC AGT C.* In this way, the Kozak sequence CCACCATGG (SEQ ID NO:4) is constructed using the bases CC in the BamHI digestion site GGATCC, the base A and an implicit sequence CCATGG of human p53 cDNA. Human p53 tumor suppressor gene coding region was amplified by a PCR reaction, wherein the template was the wild-type human p53 cDNA described above; the enzyme was *TaKaRa LA Taq*™ DNA polymerase (Bao biotech Ltd. (Dalian)); the reaction condition included: i) denaturalization at 94°C for 2min, ii) denaturalization at 94°C for 30 sec, iii) annealing at 45°C for 40 sec, iv) extension at 72°C for 60 sec, v) repeating ii)-iv) for 30 cycles, vi) extension at 72°C for 5 min, vii) storing at 4°C. A large amount of fragment of 1206bp containing human p53 gene coding fragment were obtained after the above reaction, wherein human p53 gene coding fragment as shown in SEQ ID NO:5 carries a Kozak sequence. The result of agarose gel electrophoretic analysis was shown in Figure 2.
2) Cloning and sequencing of khp53 tumor suppressor gene coding sequence:
   The PCR product (SEQ ID NO:5) of human p53 gene carrying a Kozak sequence was ligated to a T vector (Shenggong Biological Engineering and Technology Service Ltd of Shanghai) directly. The resultant was used to transform JM109 host bacteria (Fundamental medical research institute, Military medical academy of sciences of Beijing). The transformed bacteria was spread on a LB agar plate containing X-gal, IPTG and Amp. There appeared many blue and white colonies on the plate. The two colour of colonies were picked, 6 clones each, and amplified in a volume of 2ml, respectively. PCR template was prepared by thermal cleavge the resulted bacterial solution. Using that PCR template, a PCR reaction (The reaction conditions included: i)denaturalization at 94°C for 2min, ii) denaturalization at 94°C for 30 sec, iii) annealing at 50°C for 30 sec, iv) extension at 72°C for 30 sec, v) repeating ii)-iv) for 30 cycles, vi) extension at 72°C for 5 min, vii) storing at 4°C) was performed to amplify the 277bp specific fragment in human p53 tumor suppressor gene coding region, so as to determine whether human p53 tumor suppressor gene was inserted into those clones. In the PCR reaction, the upstream primer 5' -AGCACTGTCC AACAACACCA 3' (SEQ ID NO:6) and downstream primer 5' -ATA GGATCC ATG TCA GTC TGA GTC AG 3' (SEQ ID NO:2) were used. The result showed that the PCR product of the 277bp fragment didn't exist in the blue clones, meaning the blue clones were blank vectors; whereas the PCR product of the 277bp fragment did exist in the white clones. Then a small amount of plasmids identified as positive clones by PCR were extracted, digested by BamHI, and electrophoresed. A band of about 1200bp appeared in front of the band of the linear T vector, confirming there was a human p53 tumor suppressor gene coding fragment both ended with BamHI digestion sites inserted into the T vector. That type of plasmid was called pUCm-khp53, and sequenced in both directions. The sequence of human p53 tumor suppressor gene coding fragment is same as the expected result.
3) Construction of the recombinant adenovirus shuttle vector pDC515(io)-khp53:
   The plasmid pUCm-khp53 and adenovirus shuttle vector pDC515(io) were amplified, extracted and digested with BamHI. The desired gene fragments and the linear vectors were separated by agarose gel electrophoresis. After recovered by gel cutting, the purified target fragments were quantified by UV The inserted fragments and the vectors were mixed with a ratio of 3:1, and ligated at 16°C with T4 DNA ligase overnight. The resultant was used to transform competent *E*. *coli.* The positive clones were pre-screened out by the method mentioned in above part 2). The inserted direction of a target gene was determined by a PCR reaction, wherein an upstream primer 5'-ACA TCC ACT TTG CCT TTC TC 3' (SEQ ID NO:7) and a downstream primer 5'-GG GAC AGC ATC AAA TCA TCC 3'(SEQ ID NO:8) were used, and the reactive conditions included: i) denaturalization at 94°C for 2min, ii) denaturalization at 94°C for 30 sec, iii) annealing at 50°C for 30 sec, iv) extension at 72 °C for 30 sec, v) repeating ii)-iv) for 30 cycles, vi) extension at 72°C for 5 min, vii) storing at 4°C. Then a small amount of plasmids identified as positive clones by PCR (i.e. the clones with the fragment inserted in a forward direction) were extracted, digested by BamHI, and electrophoresed. A band of ~1 200bp appeared in front of the band of the linear shuttle vector, as shown in Figure 3, confirming there was a human p53 tumor suppressor gene coding fragment both ended with BamHI digestion sites inserted into the vector. Lastly, the positive clones with forwardly inserted genes, named as pDC515(io)-khp53, were stored at -20°C.
4) Preparation of the adenovirus strain comprising recombinant khp53 tumor suppressor gene:
   The plasmid of pDC515(io)-khp53, the adenoviral genomic backbone DNA i.e. pBHGfrt(del)E1,3FLP, etc were mass amplified, extracted, purified and quantified (The methods refer to Laboratory Manual of Molecular Cloning, J. Sambrook et al., 2002). After the revival, amplification and plating of 293IQ cells, the 2931Q cells were co-transfected with pDC515(io)-khp53 and the adenoviral genomic backbone DNA (total amount of DNA was 800ng; and the molar ratio was 1:1) under the induction by Lipofectamine2000 transfection reagent (Zhanchen Biotech Ltd of Guangzhou). And a negative control was set up as well. After 8 days, a cellular pathological effect (CPE) happened to 293IQ cells in the co-transfected wells, whereas the 293IQ cells in the negative control wells grew normally. Therefore, it was primarily concluded that the recombinant adenovirus was generated. In the day 10, a complete pathological change happened to 293IQ cells in the co-transfected wells, as shown in Figure 4. The pathologically changed cells and the supernatant were collected, repeatedly frozen and melted 3 times at -20°C~37°C, and stored at -20°C for later use.

### Example 2: Identification, cloning, mass amplification and purification of the adenovirus comprising recombinant khp53 tumor suppressor gene for manufacturing a clinical grade therapeutic agent

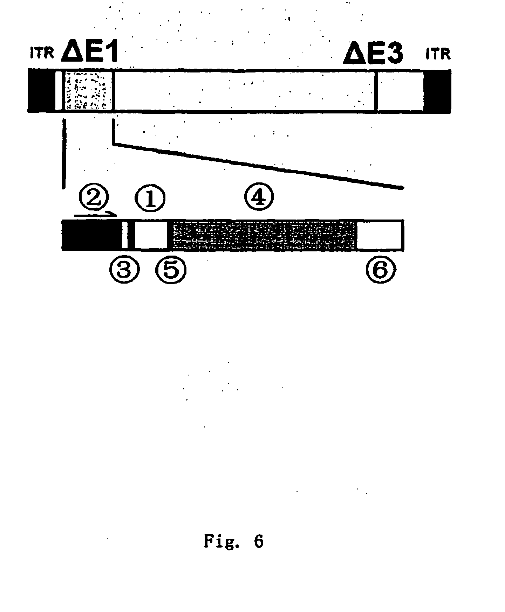

1) Identification of the adenovirus comprising recombinant khp53 tumor suppressor gene:
   A PCR reaction was carried out to amplify the template prepared by thermal cleavage of recombinant adenovirus, wherein an upstream primer 5'-TCG TTT CTC AGC AGC TGT TG 3' (SEQ ID NO:9) and a downstream primer 5' -CAT CTG AAC TCA AAG CGT GG 3' (SEQ ID NO:10) were used; the reactive conditions included: i) denaturalization at 94°C for 2min, ii) denaturalization at 94°C for 30 sec, iii) annealing at 55°C for 30 sec, iv) extension at 72°C for 40 sec, v) repeating ii)-iv) for 30 cycles, vi) extension at 72°C for 5 min, vii) storing at 4°C. If an Ad5 genomic specific fragment (11-13.4mu, 860bp) was amplified, the resultant virus described above was identified as a type 5 adenovirus. The 277bp specific fragment in human p53 tumor suppressor gene coding region was amplified by PCR to determine whether there inserted a human p53 tumor suppressor gene in said type 5 adenovirus, wherein an upstream primer 5' -AGCACTGTCC AACAACACCA 3' (SEQ ID NO:6) and a downstream primer 5' -ATA GGATCC ATG TCA GTC TGA GTC AG 3' (SEQ ID NO:2) were used. The results were shown in Figure 5, wherein all of the 6 clones were type 5 adenoviruses and all carried human p53 tumor suppressor genes.
2) Cloning of the adenovirus comprising recombinant khp53 tumor suppressor gene:
   A suspension of 293IQ cells with a concentration of 1 × 10⁵ cells/ml was prepared with MEM medium. A 96-well plate was inoculated with the suspension, 100µl/well. The diluted solution of the above recombinant adenovirus was prepared by being diluted in a 10 time series, and utilized to transfect 293IQ cells, respectively. After cultured at 37°C in a CO₂ incubator for 10 days, the cells were observed under an inverted microscope and the CPE status involving the most diluted diluent was evaluated and recorded. The mono-porous cells generating only one plaque and the supernatant were collected, repeatedly frozen and melted 3 times at -20°C ~37°C, further amplified on 293IQ cells, and used as a seed of the cloned recombinant adenovirus, with the structure shown in Figure 6. Such a recombinant adenovirus was named as "E1 and E3 defective type 5 recombinant adenovirus rAdMH-khp53", shorten as rAdMH-khp53. rAdMH-khp53 was deposited in China Center for Type Culture Collection, Wuhan, China in June 20^{th}, 2005, with a deposit number of CCTCC V200508.
3) Mass amplification and storage of a recombinant adenovirus:
   Under the condition of 37°C and 5%CO₂, 293IQ cells were cultured in 100 cell culture plates (medium: MEM+10%FBS) with Φ15cm. Twenty microliter of the above seed virus solution was not added until the cells reached 80-90% confluence. The cells were returned back to and further cultured, observed under the condition of 37°C and 5%CO₂. After pathological changes happened to all cells, the suspended cells were pipetted up and down, and the cell suspension was collected in a centrifuge tube. Cells were collected after centrifuged at 800-1000rpm for 5 min. The cell pellet collected from each 300cm² suspension were resuspended in 2ml adenoviral storage reagent (1 ×PBS²⁺+10%glycerol), repeatedly frozen and melted 3 times at -20°C~37°C, and centrifuged at 4°C, 2000rpm for 10 min. The supernatant was collected, filtered through a 0.45/0.2µm membrane, and stored at -20°C for later use.
4) Purification and preparation of a clinical grade recombinant adenovirus gene therapeutic agent by ion-exchange chromatography:
   AKTA™ explorer 100 automatic chromatographic system (Amersham Biosciences (China) Ltd.) was used. The chromatographic conditions included: a 20ml Q Sepharose XL chromatographic column, 400ml (20-column volume) elution reagent comprising 1M NaCl, 50mM Tris/HCl, pH8.0, 5% glycerol, with a linear grads from 0 to 100%. The fraction of recombinant adenoviruses was collected according to the chromatogram. Then, the column was eluted with 0.1M NaOH, and the purified recombinant adenovirus was further desalted, thereby adjusting the pH (pH 8.0). The sample was sterilized by being filtered through a 0.2µm membrane, packed and stored in a -80°C freezer in the end.

### Example 3: Assay for the titer value of a recombinant adenovirus comprising recombinant khp53 tumor suppressor gene, measurement for the number of adenovirons, and analysis of gene expression in human malignant tumor cells

1) Assay for the titer value of recombinant adenovirus by TCID50 method:
   A suspension of 293IQ cells with a concentration of 1×10⁵ cells/ml was prepared with MEM medium. A 96-well plate was inoculated with the suspension, 100µl/well. The diluted virus solution was prepared by being diluted in a 10 time series, and utilized to transfect 2931Q cells, respectively. For each row, 100µl of the diluted virus solution with the same concentration was added into the first 10 wells respectively; and the same volume of MEM containing 2%BCS was added into the 11^{th} and 12^{th} wells at the same row respectively, as negative controls. After cultured at 37°C in a CO₂ incubator for 10 days, the cells were observed under an inverted microscope and the CPE status was evaluated and recorded. Lastly, the titer value of the recombinant adenovirus was calculated in accordance with the following formula: T=10^{1+d(S-0.5)} IU/ml, wherein d=Log 10(diluted folds)=1, S=the sum of positive ratios from the first dilution (for the value of each well, positive counts for 0.1; negative counts for 0). The difference between the titer values obtained from two parallel experiments should be ≤ 10^{0.7}=5. The above formula is then shorten as T=10^{S+0.5} IU/ml. As a result, for those two 96-well plates, S values were 8.8 and 8.6, titer values were 2.00×10¹⁰IU/ml and 1.26×10¹⁰IU/ml, respectively, and the average titer value was 1.63 × 10¹⁰IU/ml.
2) Measurement of the number of adenovirons by UV absorption method:
   After addition of an equal volume of 0.2%SDS solution, 250µl recombinant adenovirus purified in step 4) of Example 2 was cleaved, mixed evenly by shacking, kept in a 56°C water bath for 10 min, and transiently centrifuged after the temperature dropped to room temperature. The mixture of viral storage solution and 0.2%SDS, with a volume ratio of 1:1 was used as a blank control. The absorbance was measured at 260nm and 280nm wavelength, respectively. The experiments were carried out twice in parallel. The number of virons was calculated in accordance with the following formula: A260xdilution foldx1.1 × 10¹². The result showed that the viron number of this batch of rAdMH-khp53 was 4.6×10¹¹VP/ml.
3) RT-PCR detection of p53 gene expression:
   Human osteosarcoma Saos-2 cells (expressing no p53 gene) and human lung cancer A549 cells (expressing a wild-type p53 gene) (Laboratorial Animal Center of Zhongshan University of Guangzhou) were revived, cultured and plated on 6cm cell culture plates (two plates for Saos-2 cells; one plate for A549 cells). The Saos-2 cells in one plate were infected with rAdMH-khp53 recombinant adenovirus at 10MOI. After 10 hours, total RNA was extracted from Saos-2, Saos-2+rAdMH-khp53 and A549 cells by Trizol reagent (invitrogen), according to the method provided in the kit. The first strand of the cDNA of Saos-2, Saos-2+rAdMH-khp53 and A549 cells were synthesized using Fermentas Rebert Aid First Strand cDNA Synthesis Kit (Shenggong Biological Engineering and Technology Service Ltd of Shanghai), according to the method provided in the kit. p53 specific fragment was amplified (the amplification method was same as the method for identifying p53 gene in the part 1) of Example 2) by PCR, wherein the first strand of the cDNA for each type of cells was used as the template. The specific fragment of house-keeping gene GAPDH amplified by PCR was used as inner control, and a blank control was set up as well. The PCR product was observed by agarose gel electrophoresis and photographed under UV, as shown in Figure 7. Lane I presented the RT-PCR result for total RNA of A549 cells, showing the expression of p53 mRNA. Lane 2 presented the RT-PCR result for total RNA of Saos-2 cells, showing no expression of p53 mRNA. Lane 3 presented the RT-PCR result for total RNA of Saos-2 cells infected with rAdMH-khp53 for 10 hours, showing the expression of p53 mRNA, which was resulted by the expression of an exogenous gene. Lane 4 presented a negative control, showing no band, which is due to the absence of template in the RT-PCR system. Lane M presented the DNA molecular weight marker. Lanes 5-7 presented the results of RT-PCR amplification with total RNA of A549, Saos-2, and Saos-2+rAdMH-khp53 cells as the template, respectively, and the specific primers for house-keeping gene GAPDH as the primers, all showing bands. Lane 8 presented the negative control, showing no band, which is due to the absence of template in the RT-PCR system.
4) Detection of P53 protein expression by SDS-PAGE and Western blotting:
   Total protein was extracted from the cells treated as part 3) using a conventional method, and then quantified. Protein electrophoresis was performed using 15% polyacrylamide gel. A piece of NC membrane and two pieces of Whatman filter paper were cut off and immerged in a transferring buffer for 5 min with two pieces of sponge. After the electrotransferring device was set up, membrane transfer was carried out under 50mA current overnight. The transferred membrane was washed by PBS for 2 min, followed by being blocked in PBS containing 5% BSA for 30 min. The membrane was incubated for 1 hour on a shaker following the addition of 5 ml of mouse anti-human P53 primary antibody (Shangbo Biological Medical Technology (Beijing) Ltd.) (1:500 diluted by PBS containing 5% BSA). The membrane was washed by PBS for 5 times, 5 min each time. Hop-Human P53 secondary antibody (Shangbo Biological Medical Technology (Beijing) Ltd.) was added and diluted 1:1000 (by PBS with 5% BSA). The membrane was incubated in the diluted secondary antibody solution for 30 min on a shaker, and washed by PBS for 5 times, 5 min each time. Then the membrane was placed into DAB color-developing agent, gently shaked at room temperature. The color reaction was observed, and the membrane was washed by water immediately after color of the bands became enough dark (1-3 min). Subsequently, the membrane was transferred into a PBS solution, and photographed after being taken out of the solution. The result was shown in Figure 8. Lane 1 presented the Western blot result for total RNA of Saos-2 cells, showing Actin protein blot, but no P53 protein blot. Lane 2 presented the result for total protein of Saos-2 cells infected with rAdMH-khp53 for 10 hours, which showed a very big P53 protein blot, indicating large amount of P53 protein expression, and an Actin protein blot with a size similar to that of Lane 1. Lane 3 presented the Western blot result for total protein of A549 cells, showing both a P53 protein blot and an Actin protein blot.

### Example 4: In vitro and in vivo growth inhibition of human malignant tumor cells by a recombinant adenovirus comprising recombinant khp53 tumor suppressor gene

1) Experiment for measuring the survival rate of cells by MTT:
   A 96-well plate was inoculated with malignant tumor cells (human lung cancer A549 cells, human liver cancer HepG2 cells, human breast cancer MCF-7 cells, human nasopharyngeal cancer HNE-1 cells, human ovarian caner SK-OV-3, human cervical cancer Hela cells, etc) (all available from the Laboratorial Animal Center of Zhongshan University of Guangzhou), respectively. After complete adhesion, the cells were infected with rAdMH (without any exogenous gene) (generated by recombination of the shuttle plasmid without any exogenous gene, i.e. pDC515(io) and the adenoviral genomic backbone, i.e. pBHGfrt(del)E1,3FLP in 293IQ cells, according to the preparation process of rAdMH-khp53 described above), rAdMH-LacZ (with a β-galactosidase gene)(generated by recombination of the shuttle plasmid with a forwardly inserted β-galactosidase gene, i.e. pDC515(io)-LacZ and the adenoviral genomic backbone, i.e. pBHGfrt(del)E1,3FLP in 293IQ cells, according to the preparation process of rAdMH-khp53 described above, wherein the recombinant plasmid pDC515(io)-LacZ was obtained as follows: digesting LacZ gene (SEQ ID NO:12) with the restriction endonulease HindIII and blunting the ends with Klenow enzyme; digesting pDC515(io) with the restriction endonulease EcoRI and blunting the ends with Klenow enzyme; digesting both of above gene fragments with the restriction endonulease BamHI; separating those two DNA fragments by electrophoresis; ligating, transforming and screening the desired plasmid after purification) and rAdMH-khp53 of different MOI (0, 2.5, 5, 10, 25, 50, 100, 250), respectively. The culture solution was removed 4 days later, and 150 µL (0.5 mg/mL) MTT solution was added into each well. The MTT solution was removed after reacting at 37°C for 4 hours, and 150 µL DMSO/well was added. The plate was kept on a horizontal shaker for 10 min. Then A value was measured at the wavelength of 570 nm. The result showed that rAdMH-khp53 possessed a clearly killing function to all malignant tumor cells. For example, in the experiment on human lung caner A549 cells, the number of survived cells in the treated group was significantly decreased, as shown in Figure 9.
2) Experiment on treatment of the transplantation tumors by rAdMH-khp53 gene therapeutic agent in a naked mouse:
   Nine naked mice were subcutaneously injected with 0.1 ml PBS suspension containing 5×10⁶ human lung cancer A549 cells at different locations on the anterior part of the back, the right posterior part of the back and the left posterior part of the back, respectively. After tumor inoculation for 6 days, 0.1 ml of 1 × 10⁹IU rAdMH-khp53 (the right posterior part of the back), rAdMH-LacZ (the anterior part of the back) and a blank control PBS (left posterior part of the back) were injected into tumor nodules at the different locations (or the locations inoculated with tumors), respectively. The injection was performed every 2 days, and 6 times in total. Volume of tumors was measured before each injection, for a period of 24 days. The result indicated that the volume of the tumors in the gene therapy group was significantly reduced, as shown in Figure 10. After the experiments, the animals were sacrificed. The organs such as tumors, hearts, livers, spleens, lungs, kidneys and brains, etc were cut off, fixed, embedded in paraffin, and sliced. An immunohistochemical analysis was performed to detect the distribution of the recombinant adenoviruses introduced in target tissues and non-target tissues in animal. The result showed that the structure of tissues such as hearts, livers, spleens, lungs, kidneys and brains, etc maintained well, and there was no clear damage in those tissues. The result also indicated that the recombinant adenoviruses existed in tumor tissues, but not in non-tumor tissues.

## Claims

1. A recombinant adenovirus shuttle vector, wherein a Kozak sequence and an exogenous gene are inserted between an operator and a PolyA signal sequence in the adenovirus shuttle vector pDC315(io) or pDC316(io), said adenovirus shuttle vector pDC315(io) is shown as Figure 11 (A); said adenovirus shuttle vector pDC316(io) is shown as Figure 11 (B), wherein said Kozak sequence is CCACCATGG and said exogenous gene is human p53 gene and the sequence thereof is as defined by SEQ ID NO:3.

2. A recombinant adenovirus shuttle vector, wherein a Kozak sequence and an exogenous gene are inserted between an operator and a PolyA signal sequence in the adenovirus shuttle vector pDC515(io) or pDC516(io), said adenovirus shuttle vector pDC515(io) is shown as Figure 11 (C); said adenovirus shuttle vector pDC516(io) is shown as Figure 11 (D), wherein said Kozak sequence is CCACCATGG and said exogenous gene is human p53 gene and the sequence thereof is as defined by SEQ ID NO:3.

3. An eukaryotic gene expression system, comprising the sequence of SEQ ID NO:11.

4. A recombinant adenovirus obtained by co-transfecting 293lQ cells comprising an E. coli LAC suppressor gene with the recombinant adenovirus shuttle vector according to any one of claim 1 or 2 and an adenoviral backbone, wherein if said adenovirus shuttle vector is pDC315(io) or pDC316(io), said adenovirus backbone is pBHGlox(delta)E1,3Cre; if said adenovirus shuttle vector is pDC515(io) or pDC516(io), said adenovirus backbone is pBHGfrt(del)E1,3FLP, said adenovirus backbone pBHGlox(delta)E1,3Cre is shown as Figure 12(A), said adenovirus backbone pBHGfrt(del)E1,3FLP is shown as Figure 12(B).

5. The recombinant adenovirus according to claim 4, which is an adenovirus with a deposite number of CCTCC V200508, wherein adenovirus shuttle vector is pDC515(io), the transgene is the wild type human p53 gene coding sequence as defined by positions 831-2012 of SEQ ID NO:11.

6. A medicament for use in treating cancers comprising the recombinant adenovirus according to any one of claim 4 or 5 and optically, a pharmaceutically acceptable excipient.

7. Use of the recombinant adenovirus according to any one of claim 4 or 5 for preparing a medicament for treating cancers.

8. The use according to claim 7, wherein said cancer is selected from lung cancer, liver cancer, breast cancer, nasopharyngeal cancer, ovarian cancer and cervical cancer.

9. A method for producing the recombinant adenovirus shuttle vector of claim 1 or 2, comprising inserting an exogenous gene linked with a Kozak sequence between an operator and a PolyA signal sequence of the adenovirus shuttle vector pDC315(io), pDC316(io), pDC515(io) or pDC516(io) in a forward direction.

10. A method for producing the recombinant adenovirus of claim 4 or 5, comprising co-transfecting 2931Q cells comprising an E. coli Lac suppressor gene with the recombinant adenovirus shuttle vector according to any one of claim 1 or 2 and an adenoviral backbone, wherein if said adenovirus shuttle vector is pDC315(io) or pDC316(io), said adenovirus backbone is pBHGlox(delta)E1,3Cre; if said adenovirus shuttle vector is pDC515(io) or pDC516(io), said adenovirus backbone is pBHGfrt(del)E-1,3FLP, said adenovirus backbone pBHGlox(delta)E1,3Cre is shown as Figure 12(A), said adenovirus backbone pBHGfrt(del)E1,3FLP is shown as Figure 12(B).

## Patentansprüche

1. Rekombinanter Adenovirus-Shuttle-Vektor, wobei eine Kozak-Sequenz und ein exogenes Gen zwischen einem Operator und einer PolyA-Signalsequenz in dem Adenovirus-Shuttle-Vektor pDC315(io) oder pDC316(io) eingefügt sind, wobei der Adenovirus-Shuttle-Vektor pDC315(io) als Figur 11 (A) gezeigt ist; wobei der Adenovirus-Shuttle-Vektor pDC316(io) als Figur 11 (B) gezeigt ist, wobei die Kozak-Sequenz CCACCATGG ist und das exogene Gen ein menschliches p53-Gen ist und die Sequenz davon wie durch SEQ ID NO:3 definiert ist.

2. Rekombinanter Adenovirus-Shuttle-Vektor, wobei eine Kozak-Sequenz und ein exogenes Gen zwischen einem Operator und einer PolyA-Signalsequenz in dem Adenovirus-Shuttle-Vektor pDC515(io) oder pDC516(io) eingefügt sind, wobei der Adenovirus-Shuttle-Vektor pDC515(io) als Figur 11 (C) und der Adenovirus-Shuttle-Vektor pDC516(io) als Figur 11 (D) gezeigt ist, wobei die Kozak-Sequenz CCACCATGG ist und das exogene Gen ein menschliches p53-Gen ist und die Sequenz davon wie durch SEQ ID NO:3 definiert ist.

3. Eukariotisches Genexpressionssystem, das die Sequenz von SEQ ID NO:11 aufweist.

4. Rekombinanter Adenovirus, erhalten durch Kotransfektion von 293IQ-Zellen, die ein E.-coli-lac-Suppressor-Gen enthalten, mit dem rekombinanten Adenovirus-Shuttle-Vektor nach Anspruch 1 oder 2 und einem adenoviralen Rückgrat, wobei das Adenovirusrückgrat pBHGlox(delta)E1,3Cre ist, wenn der Adenovirus-Shuttle-Vektor pDC315(io) oder pDC316(io) ist und wobei das Adenovirusrückgrat pBHGfrt(del)E1,3FLP ist, wenn der Adenovirus-Shuttle-Vektor pDC515(io) oder pDC516(io) ist, wobei das Adenovirusrückgrat pBHGlox(delta)E1,3Cre als Figur 12 (A) und das Adenovirusrückgrat pBHGfrt(del)E1,3FLP als Figur 12 (B) gezeigt ist.

5. Rekombinanter Adenovirus nach Anspruch 4, der ein Adenovirus mit einer Depotnummer CCTCC V200508 ist, wobei der Adenovirus-Shuttle-Vektor pCD515(io) ist, und wobei das Transgen die kodierende Sequenz des wilden menschlichen p53-Gens ist, wie sie durch die Positionen 831-2012 von SEQ ID NO:11 definiert ist.

6. Medikament zur Verwendung in der Behandlung von Krebserkrankungen, welches den rekombinanten Adenovirus nach Anspruch 4 oder 5 und wahlweise einen pharmazeutisch akzeptablen Hilfsstoff enthält.

7. Verwendung des rekombinanten Adenovirus nach Anspruch 4 oder 5 zur Herstellung eines Medikaments zur Behandlung von Krebserkrankungen.

8. Verwendung nach Anspruch 7, wobei die Krebserkrankung Lungenkrebs, Leberkrebs, Brustkrebs, Nasen-Rachen-Krebs, Eierstockkrebs oder Gebärmutterhalskrebs ist.

9. Verfahren zur Herstellung des rekombinanten Adenovirus-Shuttle-Vektors nach Anspruch 1 oder 2, wobei ein mit einer Kozak-Sequenz verbundenes exogenes Gen zwischen einem Operator und einer PolyA-Signalsequenz des Adenovirus-Shuttle-Vektors pDC315(io), pDC316(io), pDC515(io) oder pDC516(io) in einer Vorwärtsrichtung eingefügt wird.

10. Verfahren zur Herstellung des rekombinanten Adenovirus nach Anspruch 4 oder 5, wobei 293lQ-Zellen, die ein E.-coli-lac-Supressor-Gen enthalten, mit dem rekombinanten Adenovirus-Shuttle-Vektor nach Anspruch 1 oder 2 und einem adenoviralen Rückgrat kotransfektiert werden, wobei das Adenovirusrückgrat pBHGlox(delta)E1,3Cre ist, wenn der Adenovirus-Shuttle-Vektor pDC315(io) oder pDC316(io) ist, wobei das Adenovirusrückgrat pBHGfrt(del)E1,3FLP ist, wenn der Adenovirus-Shuttle-Vektor pDC515(io) oder pDC516(io) ist, wobei das Adenovirusrückgrat pBHGlox(delta)E1,3Cre als Figur 12 (A) und das Adenovirusrückgrat pBHGfrt(del)E1,3FLP als Figur 12 (B) gezeigt ist.

## Revendications

1. Vecteur navette adénoviral recombinant, dans lequel une séquence de Kozak et un gène exogène sont insérés entre un opérateur et une séquence de signal PolyA dans le vecteur navette adénoviral pDC315(io) ou pDC316(io), ledit vecteur navette adénoviral pDC315(io) est montré à la Figure 11 (A), ledit vecteur navette adénoviral pDC316(io) est montré à la Figure 11 (B), ladite séquence de Kozak est CCACCATGG et ledit gène exogène est un gène humain p53 et la séquence de celui-ci est telle que définie par SEQ ID NO:3.

2. Vecteur navette adénoviral recombinant, dans lequel une séquence de Kozak et un gène exogène sont insérés entre un opérateur et une séquence de signal PolyA dans le vecteur navette adénoviral pDC515(io) ou pDC516(io), ledit vecteur navette adénoviral pDC515(io) est montré à la Figure 11 (C); ledit vecteur navette adénoviral pDC516(io) est montré à la Figure 11 (D), ladite séquence de Kozak est CCACCATGG et ledit gène exogène est un gène humain p53 et la séquence de celui-ci est telle que définie par SEQ ID NO:3.

3. Système d'expression génétique eukariotique, comportant la séquence de SEQ ID NO:11.

4. Adénovirus recombinant, obtenu en cotransfectant des cellules 293lQ, qui comprennent un gène suppresseur d'E.-coli-lac, avec le vecteur navette adénoviral recombinant selon la revendication 1 ou 2 et une épine dorsale adénovirale, ladite épine dorsale adénovirale étant pBHGlox(delta)E1,3Cre si ledit vecteur navette adénoviral est pDC315(io) ou pDC316(io); ladite épine adénovirale étant pBHGfrt(del)E1,3FLP si ledit vecteur navette adénoviral est pDC515(io) ou pDC516(io); ladite épine dorsale adénovirale pBHGlox(delta)E1,3Cre étant montrée à la Figure 12 (A), ladite épine dorsale pBHGfrt(del)E1,3FLP étant montrée à la Figure 12 (B).

5. Adénovirus recombinant selon la revendication 4, qui est un adénovirus avec un numéro de dépôt de CCTCC V200508, le vecteur navette adénoviral étant pCD515(io), le transgène étant la séquence codante du gène humain p53 sauvage, telle que définie par les positions 831-2012 de SEQ ID NO:11.

6. Médicament pour être utilisé lors du traitement de cancer, comportant l'adénovirus recombinant selon la revendication 4 ou 5 et, de manière facultative, un excipient qui est acceptable pharmaceutiquement.

7. Utilisation de l'adénovirus recombinant selon la revendication 4 ou 5, pour préparer un médicament de traitement de cancer.

8. Utilisation selon la revendication 7, ledit cancer étant un cancer du poumon, un cancer du foie, un cancer du sein, un cancer du rhinopharynx, un cancer de l'ovaire ou un cancer du col de l'utérus.

9. Procédé pour produire le vecteur navette adénoviral recombinant selon la revendication 1 ou 2, dans lequel un gène exogène lié à une séquence de Kozak est inséré entre un opérateur et une séquence de signal PolyA du vecteur navette adénoviral pDC315(io), pDC316(io), pDC515(io) ou pDC516(io) dans une direction d'avant.

10. Procédé pour produire un adénovirus recombinant selon la revendication 4 ou 5, dans lequel des cellules 2931Q comportant un gène suppresseur d'E.-coli-lac sont cotransfectées avec le vecteur navette adénoviral recombinant selon la revendication 1 ou 2 et une épine dorsale adénovirale, ladite épine dorsale adénovirale étant pBHGlox(delta)E1,3Cre si ledit vecteur navette adénoviral est pDC315(io) ou pDC316(io); ladite épine dorsale adénovirale étant pBHGfrt(del)E1,3FLP si ledit vecteur navette adénoviral est pDC515(io) ou pDC516(io), ladite épine dorsale adénovirale pBHGlox(delta)E1,3Cre étant montrée à la Figure 12 (A), ladite épine dorsale adénovirale pBHGfrt(del)E1,3FLP étant montrée à la Figure 12 (B).


### Sequence Listing

<110> Qing Jun XIE
<120> A recombinant adenovirus comprising recombinant khp53 gene and preparation method and uses thereof
<130> FP090022EP
<160> 12
<170> PatentIn version 3.1
<210> 1
   <211> 29
   <212> DNA
   <213> Artificial Sequence
<400> 1
   ataggatcca ccatggagga gccgcagtc 29
<210> 2
   <211> 26
   <212> DNA
   <213> Artificial Sequence
<400> 2
   ataggatcca tgtcagtctg agtcag 26
<210> 3
   <211> 1187
   <212> DNA
   <213> Human
<400> 3
   ccatggagga gccgcagtca gatcctagcg tcgagccccc tctgagtcag gaaacatttt 60
   cagacctatg gaaactactt cctgaaaaca acgttctgtc ccccttgccg tcccaagcaa 120
   tggatgattt gatgctgtcc ccggacgata ttgaacaatg gttcactgaa gacccaggtc    180
   cagatgaagc tcccagaatg ccagaggctg ctccccgcgt ggcccctgca ccagcagctc    240
   ctacaccggc ggcccctgca ccagccccct cctggcccct gtcatcttct gtcccttccc     300
   agaaaaccta ccagggcagc tacggtttcc gtctgggctt cttgcattct gggacagcca    360
   agtctgtgac ttgcacgtac tcccctgccc tcaacaagat gttttgccaa ctggccaaga    420
   cctgccctgt gcagctgtgg gttgattcca cacccccgcc cggcacccgc gtccgcgcca    480
   tggccatcta caagcagtca cagcacatga cggaggttgt gaggcgctgc ccccaccatg    540
   agcgctgctc agatagcgat ggtctggccc ctcctcagca tcttatccga gtggaaggaa    600
   atttgcgtgt ggagtatttg gatgacagaa acacttttcg acatagtgtg gtggtgccct    660
   atgagccgcc tgaggttggc tctgactgta ccaccatcca ctacaactac atgtgtaaca    720
   gttcctgcat gggcggcatg aaccggaggc ccatcctcac catcatcaca ctggaagact    780
   ccagtggtaa tctactggga cggaacagct ttgaggtgca tgtttgtgcc tgtcctggga    840
   gagaccggcg cacagaggaa gagaatctcc gcaagaaagg ggagcctcac cacgagctgc    900
   ccccagggag cactaagcga gcactgtcca acaacaccag ctcctctccc cagccaaaga    960
   agaaaccact ggatggagaa tatttcaccc ttcagatccg tgggcgtgag cgcttcgaga   1020
   tgttccgaga gctgaatgag gccttggaac tcaaggatgc ccaggctggg aaggagccag   1080
   gggggagcag ggctcactcc agccacctga agtccaaaaa gggtcagtct acctcccgcc   1140
   ataaaaaact catgttcaag acagaagggc ctgactcaga ctgacat                 1187
<210> 4
   <211> 9
   <212> DNA
   <213> Artificial Sequence
<400> 4
   ccaccatgg 9
<210> 5
   <211> 1187
   <212> DNA
   <213> Artificial Sequence
<400> 5
   ccaccatgga ggagccgcag tcagatccta gcgtcgagcc ccctctgagt caggaaacat     60
   tttcagacct atggaaacta cttcctgaaa acaacgttct gtcccccttg ccgtcccaag    120
   caatggatga tttgatgctg tccccggacg atattgaaca atggttcact gaagacccag    180
   gtccagatga agctcccaga atgccagagg ctgctccccg cgtggcccct gcaccagcag 240
   ctcctacacc ggcggcccct gcaccagccc cctcctggcc cctgtcatct tctgtccctt 300
   cccagaaaac ctaccagggc agctacggtt tccgtctggg cttcttgcat tctgggacag 360
   ccaagtctgt gacttgcacg tactcccctg ccctcaacaa gatgttttgc caactggcca 420
   agacctgccc tgtgcagctg tgggttgatt ccacaccccc gcccggcacc cgcgtccgcg 480
   ccatggccat ctacaagcag tcacagcaca tgacggaggt tgtgaggcgc tgcccccacc 540
   atgagcgctg ctcagatagc gatggtctgg cccctcctca gcatcttatc cgagtggaag 600
   gaaatttgcg tgtggagtat ttggatgaca gaaacacttt tcgacatagt gtggtggtgc 660
   cctatgagcc gcctgaggtt ggctctgact gtaccaccat ccactacaac tacatgtgta 720
   acagttcctg catgggcggc atgaaccgga ggcccatcct caccatcatc acactggaag 780
   actccagtgg taatctactg ggacggaaca gctttgaggt gcatgtttgt gcctgtcctg 840
   ggagagaccg gcgcacagag gaagagaatc tccgcaagaa aggggagcct caccacgagc 900
   tgcccccagg gagcactaag cgagcactgt ccaacaacac cagctcctct ccccagccaa 960
   agaagaaacc actggatgga gaatatttca cccttcagat ccgtgggcgt gagcgcttcg 1020
   agatgttccg agagctgaat gaggccttgg aactcaagga tgcccaggct gggaaggagc 1080
   caggggggag cagggctcac tccagccacc tgaagtccaa aaagggtcag tctacctccc 1140
   gccataaaaa actcatgttc aagacagaag ggcctgactc agactga 1187
<210> 6
   <211> 20
   <212> DNA
   <213> Artificial Sequence
<400> 6
   agcactgtcc aacaacacca 20
<210> 7
   <211> 20
   <212> DNA
   <213> Artificial Sequence
<400> 7
   acatccactt tgcctttctc *2*0
<210> 8
   <211> 20
   <212> DNA
   <213> Artificial Sequence
<400> 8
   gggacagcat caaatcatcc 20
<210> 9
   <211> 20
   <212> DNA
   <213> Artificial Sequence
<400> 9
   tcgtttctca gcagctgttg 20
<210> 10
   <211> 20
   <212> DNA
   <213> Artificial Sequence
<400> 10
   catctgaact caaagcgtgg 20
<210> 11
   <211> 2192
   <212> DNA
   <213> Artificial Sequence
<220>
   <221> promoter
   <222> (1).. (555)
   <223>
<220>
   <221> polyA signal
   <222> (2033).. (2191)
   <223>
<220>
   <221> Humanp53 gene
   <222> (831).. (2012)
   <223>
<220>
   <221> misc_feature
   <222> (826).. (834)
   <223> Kozak sequence
<220>
   <221> intron
   <222> (586).. (795)
   <223>
<220>
   <221> operator
   <222> (481).. (518)
   <223>
<400> 11
   gagtcattag ggactttcca atgggttttg cccagtacat aaggtcaata ggggtgaatc      60
   aacaggaaag tcccattgga gccaagtaca ctgagtcaat agggactttc cattgggttt     120
   tgcccagtac aaaaggtcaa tagggggtga gtcaatgggt ttttcccatt attggcacgt     180
   acataaggtc aataggggtg agtcattggg tttttccagc catttaatta aaacgccatg    240
   tactttccca ccattgacgt caatgggcta ttgaaactaa tgcaacgtga cctttaaacg     300
   gtactttccc atagctgatt aatgggaaag taccgttctc gagccaatac acgtcaatgg     360
   gaagtgaaag ggcagccaaa acgtaacacc gccccggttt tcccctggaa attccatatt     420
   ggcactcatt ctattggctg agctgcgttc tacgtgggta taagaggcgc gaccagcgtc     480
   ggtaccgtcg gaattgtgag cggataacaa ttggtaccgt cgcagtcttc ggtctgacaa     540
   cgcagatcga attgggcgtc taaccagtca cagtcgcaag gtaggctgag caccgtggcc     600
   accgtagggg cggcagcggg tggcggtcgg ggttgtttct ggcggaggtg ctgctgatga     660
   tgtaattaaa gtaggcggtc ttgagacggc ggatggtcga ggtgaggtgt ggcaggcttg     720
   agatcgatct ggccatacac ttgagtgaca atgacatcca ctttgccttt ctctccacag     780
   gtgtccactc ccaggtccaa ccgaattcaa gctgctagca aggatccacc atggaggagc     840
   cgcagtcaga tcctagcgtc gagccccctc tgagtcagga aacattttca gacctatgga 900
   aactacttcc tgaaaacaac gttctgtccc ccttgccgtc ccaagcaatg gatgatttga 960
   tgctgtcccc ggacgatatt gaacaatggt tcactgaaga cccaggtcca gatgaagctc 1020
   ccagaatgcc agaggctgct ccccgcgtgg cccctgcacc agcagctcct acaccggcgg 1080
   cccctgcacc agccccctcc tggcccctgt catcttctgt cccttcccag aaaacctacc 1140
   agggcagcta cggtttccgt ctgggcttct tgcattctgg gacagccaag tctgtgactt 1200
   gcacgtactc ccctgccctc aacaagatgt tttgccaact ggccaagacc tgccctgtgc 1260
   agctgtgggt tgattccaca cccccgcccg gcacccgcgt ccgcgccatg gccatctaca 1320
   agcagtcaca gcacatgacg gaggttgtga ggcgctgccc ccaccatgag cgctgctcag 1380
   atagcgatgg tctggcccct cctcagcatc ttatccgagt ggaaggaaat ttgcgtgtgg 1440
   agtatttgga tgacagaaac acttttcgac atagtgtggt ggtgccctat gagccgcctg 1500
   aggttggctc tgactgtacc accatccact acaactacat gtgtaacagt tcctgcatgg 1560
   gcggcatgaa ccggaggccc atcctcacca tcatcacact ggaagactcc agtggtaatc 1620
   tactgggacg gaacagcttt gaggtgcatg tttgtgcctg tcctgggaga gaccggcgca 1680
   cagaggaaga gaatctccgc aagaaagggg agcctcacca cgagctgccc ccagggagca 1740
   ctaagcgagc actgtccaac aacaccagct cctctcccca gccaaagaag aaaccactgg 1800
   atggagaata tttcaccctt cagatccgtg ggcgtgagcg cttcgagatg ttccgagagc 1860
   tgaatgaggc cttggaactc aaggatgccc aggctgggaa ggagccaggg gggagcaggg 1920
   ctcactccag ccacctgaag tccaaaaagg gtcagtctac ctcccgccat aaaaaactca 1980
   tgttcaagac agaagggcct gactcagact gacatggatc cagcttgtcg acttcgagca 2040
   acttgtttat tgcagcttat aatggttaca aataaagcaa tagcatcaca aatttcacaa 2100
   ataaagcatt tttttcactg cattctagtt gtggtttgtc caaactcatc aatgtatctt 2160
   atcatgtctg gatcgtctag catcgaagat cc 2192
<210> 12
   <211> 3743
   <212> DNA
   <213> Artificial Sequence
<220>
   <221> misc_feature
   <222> (1).. (6)
   <223> HindIII site
<220>
   <221> misc_feature
   <222> (3738).. (3743)
   <223> BamHI site
<400> 12
   aagcttggga tctctataat ctcgcgcaac ctattttccc ctcgaacact ttttaagccg 60
   tagataaaca ggctgggaca cttcacatga gcgaaaaata catcgtcacc tgggacatgt 120
   tgcagatcca tgcacgtaaa ctcgcaagcc gactgatgcc ttctgaacaa tggaaaggca 180
   ttattgccgt aagccgtggc ggtctggtac cggtgggtga agaccagaaa cagcacctcg 240
   aactgagccg cgatattgcc cagcgtttca acgcgctgta tggcgagatc gatcccgtcg 300
   ttttacaacg tcgtgactgg gaaaaccctg gcgttaccca acttaatcgc cttgcagcac 360
   atcccccttt cgccagctgg cgtaatagcg aagaggcccg caccgatcgc ccttcccaac 420
   agttgcgcag cctgaatggc gaatggcgct ttgcctggtt tccggcacca gaagcggtgc 480
   cggaaagctg gctggagtgc gatcttcctg aggccgatac tgtcgtcgtc ccctcaaact 540
   ggcagatgca cggttacgat gcgcccatct acaccaacgt gacctatccc attacggtca 600
   atccgccgtt tgttcccacg gagaatccga cgggttgtta ctcgctcaca tttaatgttg 660
   atgaaagctg gctacaggaa ggccagacgc gaattatttt tgatggcgtt aactcggcgt 720
   ttcatctgtg gtgcaacggg cgctgggtcg gttacggcca ggacagtcgt ttgccgtctg 780
   aatttgacct gagcgcattt ttacgcgccg gagaaaaccg cctcgcggtg atggtgctgc 840
   gctggagtga cggcagttat ctggaagatc aggatatgtg gcggatgagc ggcattttcc 900
   gtgacgtctc gttgctgcat aaaccgacta cacaaatcag cgatttccat gttgccactc 960
   gctttaatga tgatttcagc cgcgctgtac tggaggctga agttcagatg tgcggcgagt 1020
   tgcgtgacta cctacgggta acagtttctt tatggcaggg tgaaacgcag gtcgccagcg 1080
   gcaccgcgcc tttcggcggt gaaattatcg atgagcgtgg tggttatgcc gatcgcgtca 1140
   cactacgtct gaacgtcgaa aacccgaaac tgtggagcgc cgaaatcccg aatctctatc 1200
   gtgcggtggt tgaactgcac accgccgacg gcacgctgat tgaagcagaa gcctgcgatg 1260
   tcggtttccg cgaggtgcgg attgaaaatg gtctgctgct gctgaacggc aagccgttgc 1320
   tgattcgagg cgttaaccgt cacgagcatc atcctctgca tggtcaggtc atggatgagc 1380
   agacgatggt gcaggatatc ctgctgatga agcagaacaa ctttaacgcc gtgcgctgtt 1440
   cgcattatcc gaaccatccg ctgtggtaca cgctgtgcga ccgctacggc ctgtatgtgg 1500
   tggatgaagc caatattgaa acccacggca tggtgccaat gaatcgtctg accgatgatc 1560
   cgcgctggct accggcgatg agcgaacgcg taacgcgaat ggtgcagcgc gatcgtaatc 1620
   acccgagtgt gatcatctgg tcgctgggga atgaatcagg ccacggcgct aatcacgacg 1680
   cgctgtatcg ctggatcaaa tctgtcgatc cttcccgccc ggtgcagtat gaaggcggcg 1740
   gagccgacac cacggccacc gatattattt gcccgatgta cgcgcgcgtg gatgaagacc 1800
   agcccttccc ggctgtgccg aaatggtcca tcaaaaaatg gctttcgcta cctggagaga 1860
   cgcgcccgct gatcctttgc gaatacgccc acgcgatggg taacagtctt ggcggtttcg 1920
   ctaaatactg gcaggcgttt cgtcagtatc cccgtttaca gggcggcttc gtctgggact 1980
   gggtggatca gtcgctgatt aaatatgatg aaaacggcaa cccgtggtcg gcttacggcg 2040
   gtgattttgg cgatacgccg aacgatcgcc agttctgtat gaacggtctg gtctttgccg 2100
   accgcacgcc gcatccagcg ctgacggaag caaaacacca gcagcagttt ttccagttcc 2160
   gtttatccgg gcaaaccatc gaagtgacca gcgaatacct gttccgtcat agcgataacg 2220
   agctcctgca ctggatggtg gcgctggatg gtaagccgct ggcaagcggt gaagtgcctc 2280
   tggatgtcgc tccacaaggt aaacagttga ttgaactgcc tgaactaccg cagccggaga 2340
   gcgccgggca actctggctc acagtacgcg tagtgcaacc gaacgcgacc gcatggtcag 2400
   aagccgggca catcagcgcc tggcagcagt ggcgtctggc ggaaaacctc agtgtgacgc 2460
   tccccgccgc gtcccacgcc atcccgcatc tgaccaccag cgaaatggat ttttgcatcg 2520
   agctgggtaa taagcgttgg caatttaacc gccagtcagg ctttctttca cagatgtgga 2580
   ttggcgataa aaaacaactg ctgacgccgc tgcgcgatca gttcacccgt gcaccgctgg 2640
   ataacgacat tggcgtaagt gaagcgaccc gcattgaccc taacgcctgg gtcgaacgct 2700
   ggaaggcggc gggccattac caggccgaag cagcgttgtt gcagtgcacg gcagatacac 2760
   ttgctgatgc ggtgctgatt acgaccgctc acgcgtggca gcatcagggg aaaaccttat 2820
   ttatcagccg gaaaacctac cggattgatg gtagtggtca aatggcgatt accgttgatg 2880
   ttgaagtggc gagcgataca ccgcatccgg cgcggattgg cctgaactgc cagctggcgc    2940
   aggtagcaga gcgggtaaac tggctcggat tagggccgca agaaaactat cccgaccgcc    3000
   ttactgccgc ctgttttgac cgctgggatc tgccattgtc agacatgtat accccgtacg 3060
   tcttcccgag cgaaaacggt ctgcgctgcg ggacgcgcga attgaattat ggcccacacc 3120
   agtggcgcgg cgacttccag ttcaacatca gccgctacag tcaacagcaa ctgatggaaa 3180
   ccagccatcg ccatctgctg cacgcggaag aaggcacatg gctgaatatc gacggtttcc 3240
   atatggggat tggtggcgac gactcctgga gcccgtcagt atcggcggaa ttccagctga 3300
   gcgccggtcg ctaccattac cagttggtct ggtgtcaaaa ataataataa ccgggcaggc 3360
   catgtctgcc cgtatttcgc gtaaggaaat ccattatgta ctatttaaaa aacacaaact 3420
   tttggatgtt cggtttattc tttttctttt acttttttat catgggagcc tacttcccgt 3480
   ttttcccgat ttggctacat gacatcaacc atatcagcaa aagtgatacg ggtattattt 3540
   ttgccgctat ttctctgttc tcgctattat tccaaccgct gtttggtctg ctttctgaca 3600
   aactcggaac ttgtttattg cagcttataa tggttacaaa taaagcaata gcatcacaaa 3660
   tttcacaaat aaagcatttt tttcactgca ttctagttgt ggtttgtcca aactcatcaa 3720
   tgtatcttat catgtctgga tcc 3743